# EUROPÄISCHE PATENTANMELDUNG

(11) **EP 0 875 500 A2**
(43) Veröffentlichungstag der Anmeldung: **04.11.1998**
(21) Anmeldenummer: 98107217.6
(22) Anmeldetag: 21.04.1998
(51) Int. Cl.: C07C 233/18, C07C 233/20, C11D 1/52, A61K 31/16, A61K 7/06

(54) **Quaternierte Fettsäureamidoaminethoxylate**

(30) Priorität: 30.04.1997 DE 19718188; 05.06.1997 DE 19723605
(71) Anmelder: Henkel Kommanditgesellschaft auf Aktien, 40589 Düsseldorf-Holthausen (DE)
(72) Erfinder: Bigorra, Joaquin, Dr., 08203 Sabadell (ES); Pi Subirana, Rafael, Dr., 08400 Granollers (ES); Bonastre Gilabert, Nuria, Dr., 08210 Barberá del Vallés (ES)

(57) **Zusammenfassung**

Es werden neue quaternierte Fettsäureamidoaminethoxylate der Formel **(I)** vorgeschlagen, 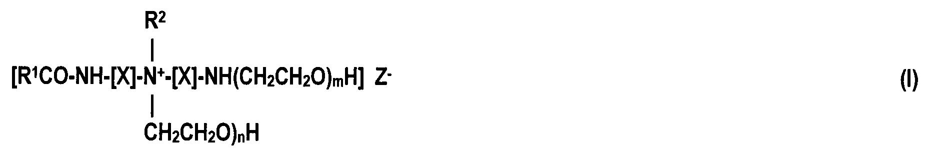 in der R¹CO für einen linearen oder verzweigten, gesättigten und/oder ungesättigten Acylrest mit 6 bis 22 Kohlenstoffatomen, R² für für einen Alkylrest mit 1 bis 4 Kohlenstoffatomen oder einen CH₂COOY-Rest, die Summe (m+n) für Zahlen von 1 bis 20 und q für Zahlen von 1 bis 5, X für eine lineare oder verzweigte Alkylengruppe mit 1 bis 6 Kohlenstoffatomen oder eine CH₂CH₂-(NH-CH₂CH₂)_{q}-Gruppe, Y für Wasserstoff oder ein Alkalimetall und Z für Halogenid oder Alkylsulfat steht, bei dem man
(a) Fettsäuren mit Di- bzw. Oligoaminen kondensiert,
(b) die resultierenden Fettsäureamidoamine an sich bekannter Weise mit Ethylenoxid umsetzt und
(c) die resultierenden ethoxylierten Fettsäureamidoamine anschließend in an sich bekannter Weise mit Alkylierungsmitteln quaterniert.
Die neuen kationischen bzw. amphoteren Tenside eignen sich zur Herstellung einer Vielzahl von oberflächenaktiven Mitteln.

## Beschreibung

### Gebiet der Erfindung

Die Erfindung betrifft quaternierte Fettsäureamidoaminethoxylate, sogenannte Amidetherquats, die man durch Kondensation von Fettsäuren mit Oligoaminen, nachfolgende Ethoxylierung und Quaternierung erhält, ein Verfahren zu ihrer Herstellung sowie ihre Verwendung zur Herstellung oberflächenaktiver Mittel.

### Stand der Technik

In den letzten Jahren haben kationische Tenside, die über hydrolysierbare Gruppen verfügen und somit leichter biologisch abbaubar sind, konventionelle Tetraalkylammoniumsalze zu einem großen Teil substituiert. Nichtsdestotrotz besteht im Markt ein ständiges Bedürfnis nach Kationtensiden mit weiter verbesserten ökotoxikologischen und anwendungstechnischen Eigenschaften, die in den unterschiedlichsten Einsatzgebieten überdurchschnittlich hohe Performance leisten sollen.

In diesem Zusammenhang sind aus der europäischen Patentschrift **EP-B1 0652931** (Henkel) Detergensgemische bekannt, die als kationische Tenside quaternierte Ester von Fettsäuren mit ethoxylierten Alkanolaminen enthalten. In der US-Patentschrift **US 5,133,885** (Colgate) wird der Einsatz von ethoxylierten Fettsäureamidoaminen zusammen mit kationischen Tensiden beschrieben.

Nachdem die Produkte des Handels jedoch nicht völlig zufriedenstellend sind, hat die komplexe Aufgabe der Erfindung darin bestanden, neue kationische bzw. amphotere Tenside zur Verfügung zu stellen, die nicht nur gut biologisch abbaubar und ausgezeichnet hautkosmetisch verträglich sind, sondern zudem noch über gute Avivage- und Antistatikeigenschaften sowie ein hohes Schaum- und Reingungsvermögen verfügen.

### Beschreibung der Erfindung

Gegenstand der Erfindung sind quaternierte Fettsäureamidoaminethoxylate der Formel **(I)**, 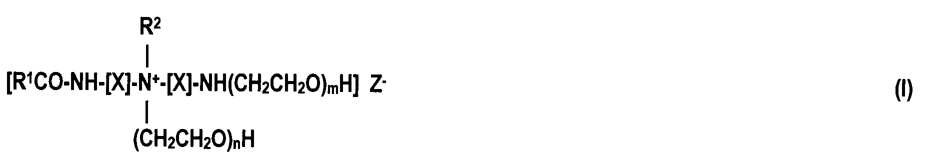 in der R¹CO für einen linearen oder verzweigten, gesättigten und/oder ungesättigten Acylrest mit 6 bis 22 Kohlenstoffatomen, R² für für einen Alkylrest mit 1 bis 4 Kohlenstoffatomen oder einen CH₂COOY-Rest, die Summe (m+n) für Zahlen von 1 bis 20 und q für Zahlen von 1 bis 5, X für eine lineare oder verzweigte Alkylengruppe mit 1 bis 6 Kohlenstoffatomen oder eine CH₂CH₂-(NH-CH₂CH₂)_{q}-Gruppe, Y für Wasserstoff oder ein Alkalimetall und Z für Halogenid oder Alkylsulfat steht.

Überraschenderweise wurde gefunden, daß die quaternierten Fettsäureamidoaminethoxylate der Erfindung das komplexe Anforderungsprofil in ausgezeichneter Weise erfüllen. Sie sind infolge der Amidbindungen leicht biologisch abbaubar und weisen eine hohe hautkosmetische Verträglichkeit auf. Kationische Tenside dieses Typs verleihen Textilien und Haaren einen angenehmen Weichgriff und erniedrigen die elektrostatische Aufladung sowohl zwischen synthetischen als auch natürlichen Fasern, was auch Keratinfasern miteinschließt; zudem besitzen sie eine ungewöhnlich hohe Aniontensidverträglichkeit. Betaine auf Basis der Fettsäureamidoaminethoxylate sind schaumstark und besitzen ein hohes Reinigungsvermögen. Sie vermögen zudem die Hautverträglichkeit anionischer Tenside zu verbessern.

Ein weiterer Gegenstand der Erfindung betrifft ein Verfahren zur Herstellung von quaternierten Fettsäureamidoaminethoxylaten der Formel **(I)**, 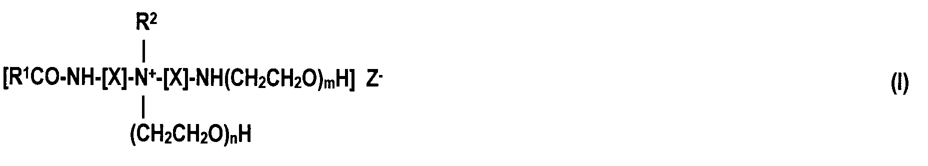 in der R¹CO für einen linearen oder verzweigten, gesättigten und/oder ungesättigten Acylrest mit 6 bis 22 Kohlenstoffatomen, R² für für einen Alkylrest mit 1 bis 4 Kohlenstoffatomen oder einen CH₂COOY-Rest, die Summe (m+n) für Zahlen von 1 bis 20 und q für Zahlen von 1 bis 5, X für eine lineare oder verzweigte Alkylengruppe mit 1 bis 6 Kohlenstoffatomen oder eine CH₂CH₂-(NH-CH₂CH₂)_{q}-Gruppe, Y für Wasserstoff oder ein Alkalimetall und Z für Halogenid oder Alkylsulfat steht, bei dem man
(a) Fettsäuren mit Di- bzw. Oligoaminen kondensiert,
(b) die resultierenden Fettsäureamidoamine an sich bekannter Weise mit Ethylenoxid umsetzt und
(c) die resultierenden ethoxylierten Fettsäureamidoamine anschließend in an sich bekannter Weise mit Alkylierungsmitteln quaterniert.

### Fettsäuren

Fettsäuren, die im Sinne der Erfindung als Ausgangsstoffe in Betracht kommen, folgen der Formel **(II)**, 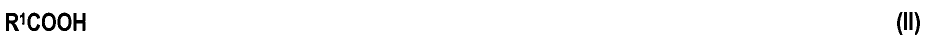 für einen linearen oder verzweigten, gesättigten und/oder ungesättigten Acylrest mit 6 bis 22, vorzugsweise 12 bis 18 Kohlenstoffatomen steht. Typische Beispiele sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen wie beispielsweise gehärtete oder partiell gehärtete Palm- oder Talgfettsäure.

### Di- bzw. Oligoamine

Geeignete Diamine bzw. Oligoamine, mit denen die Fettsäuren kondensiert werden, folgen beispielsweise der Formel **(III)**, 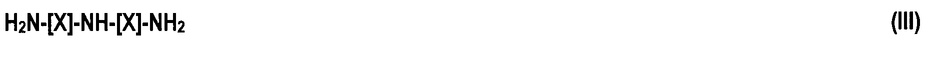 in der X für eine lineare oder verzweigte Alkylengruppe mit 1 bis 6, vorzugsweise 2 oder 3 Kohlenstoffatomen oder eine CH₂CH₂-(NH-CH₂CH₂)_{q}-Gruppe und q für Zahlen von 1 bis 5, vorzugsweise 1 bis 3 steht. Typische Beispiele für geeignete Diamine sind Ethylendiamin und Propylendiamin, als Oligoamine kommen beispielsweise Diethylentriamin, Triethylentetramin, Dipropylentriamin und Tripro-pylentetramin sowie deren Gemische in Frage. Üblicherweise setzt man die Fettsäuren und die Di- bzw. Oligoamine im molaren Verhältnis 1,3 : 1 bis 2,3 : 1, insbesondere 1,8 : 1 bis 2,2 : 1 einsetzt. Vorzugsweise setzt man die Fettsäuren und die Amine im molaren Verhältnis von etwa 2 : 1 ein, da sich der Einsatz von Diamiden, also kationischen bzw. amphoteren Tensiden mit zwei Fettresten, als besonders vorteilhaft erwiesen hat.

### Ethoxylierung

Die Ethoxylierung der Fettsäureamide folgt den Regeln der Statistik und findet überwiegend an den Amidstickstoffen statt, d.h. die Ethyelenoxideinheiten werden in die Bindung zwischen Stickstoff und acidem Proton eingefügt. Üblicherweise werden die die Fettsäureamidoamine mit durchschnittlich 1 bis 20, vorzugsweise 2 bis 10 Mol Ethylenoxid pro Mol Amid umgesetzt. Es hat sich als vorteilhaft erwiesen, die Reaktion in einem Autoklaven unter autogenem Druck (1 bis 5 bar) und bei Temperaturen im Bereich von 100 bis 150°C durchzuführen. Üblicherweise reicht die im Amid enthaltene restliche freie Fettsäure aus, um die Reaktion zu katalysieren, falls gewünscht, kann aber auch in Gegenwart entsprechender Mengen vorzugsweise alkalischer Katalysatoren wie beispielsweise Natriumhydroxid, Natriummethylat, Kaliumhydroxid, Kalium-tert.butylat, Erdalkalisalzen, Hydrotacliten und dergleichen gearbeitet werden. Die Durchführung der Reaktion erfolgt in an sich bekannter Weise. Es ist jedoch zu empfehlen, nach Beendigung der Ethoxylierung eine Nachreaktion durchzuführen, bei der man das Ethoxylat vor dem Entspannen noch ewa 30 bis 60 min bei der Reaktionstemperatur rührt. Gegebenenfalls sollte der Katalysator vor der Weiterverarbeitung neutralisiert werden.

### Quaternierung

Auch die Quaternierung der ethoxylierten Fettsäureamidoamine erfolgt in an sich bekannter Weise. Üblicherweise werden die Amidethoxylate in einem geeigneten Lösungsmittel, vorzugsweise Isopropylalkohol, daneben aber auch Wasser, Ethylenglycol, Propylenglycol oder deren Gemische gelöst bzw. dispergiert. Man kann die Quaternierung jedoch auch in Gegenwart von geeigneten Dispergatoren bzw. Emulgatoren durchführen, die im Gegensatz zu den Lösungsmitteln nicht im Anschluß abgetrennt werden, sondern im Produkt verbleiben. Geeignete Dispergatoren bzw. Emulgatoren sind insbesondere Fettalkohole mit 6 bis 22, vorzugsweise 12 bis 18 Kohlenstoffatomen sowie deren Addukte mit 1 bis 10 Mol Ethylenoxid, Partialglyceride von Fettsäuren mit 6 bis 22, vorzugsweise 12 bis 18 Kohlenstoffatomen sowie ebenfalls deren Addukte mit 1 bis 10 Mol Ethylenoxid, Alkylglucoside und dergleichen.

Als Quaternierungsmittel zur Herstellung kationischer Tenside kommen Methylhalogenide, wie beispielsweise Methylchlorid oder Dialkylsulfate, wie Dimethylsulfat oder Diethylsulfat in Betracht. Für die Herstellung amphoterer Tenside werden üblicherweise Halogencarbonsäuren bzw. deren Alkalisalze, vorzugsweise Natriumchloracetat eingesetzt. Es hat sich als vorteilhaft erwiesen, die Quaternierungsmittel bezogen auf die zu quaternierenden Gruppen im leichten Unterschuß, also beispielsweise im molaren Verhältnis 1 : 0,9 bis 1 : 0,95 bei Temperaturen im Bereich von 50 bis 90, vorzugsweise 75 bis 85°C einzusetzen, um sicherzustellen, daß die Endprodukte praktisch frei von nicht umgesetztem Alkylierungsmittel sind. Falls erforderlich, können die Quaternierungsprodukte auch mit Ammoniak, Aminen, Alkanolaminen oder Aminosäuren, vorzugsweise Glycin nachbehandelt werden.

### Herstellung hochreiner Produkte

Im Zuge der Amidierung von Fettsäuren mit Oligoaminen kann es zur Bildung von Amiden kommen, die sich von der primären (I) und von der sekundären Amingruppe (II) ableiten. Die Reaktion sei an folgendem Schaubild verdeutlicht:

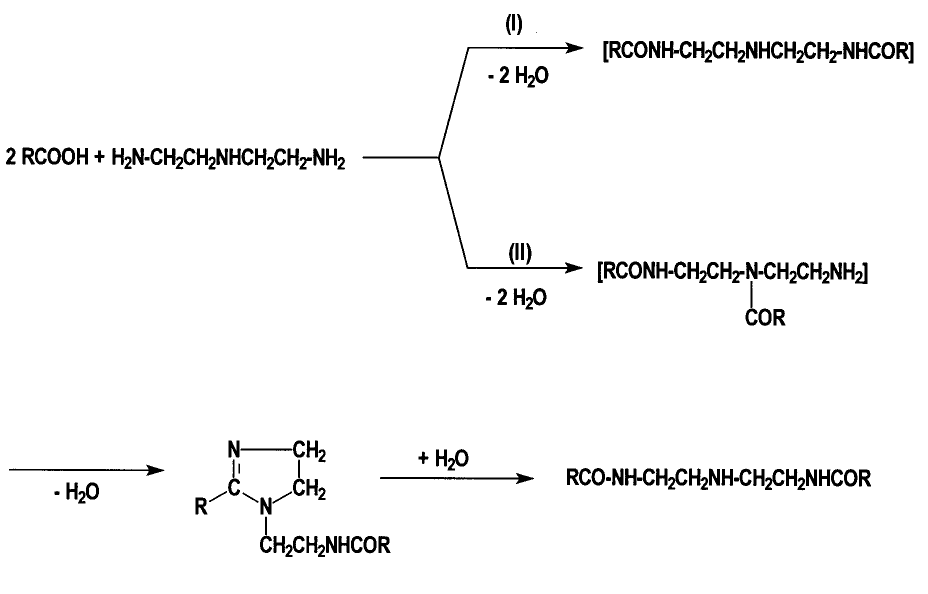

Nachdem die Bildung von sekundären Produkten aus anwendungstechnischer Sicht eher unvorteilhaft ist, besteht eine besondere Ausführungsform der Erfindung darin, sekundäre Amidoamine noch vor der Ethoxylierung durch Imidazolinbildung und nachfolge Ringöffnung, d.h. Hydrolyse mit Wasser, zu zerstören. Es resultierenden zu mehr als 90 % lineare Produkte, die wie oben angegeben weiterverarbeitet werden können. Die Imidazolinbildung erzwingt man, indem das Fettsäureamidoamin unter Anlegen eines Vakuums im Bereich von 1 bis 50 mbar bei 160 bis 220°C über einen Zeitraum von 30 bis 120 min gerührt wird. Anschließend wird mit Wasser, gegebenenfalls mit einem Überschuß von bis zu 10 Mol bezogen auf das Imidazolin, bei 50 bis 90°C über einen Zeitraum von 1 bis 12, vorzugsweise 5 bis 9 h hydrolysiert. Es empfiehlt sich, das Hydrolyseprodukt vor der Ethoxylierung zu trocknen.

### Gewerbliche Anwendbarkeit

Die neuen quaternierten Fettsäureamidoaminethoxylate erniedrigen die Grenzflächenspannung des Wassers und fördern die Vermischung ansonsten nicht miteinander mischbarer Phasen. Kationische Quaternierungsprodukte zeichnen sich durch eine besondere Haar- und Faseravivage aus und reduzieren die elektrostatische Aufladung sowohl zwischen synthetischen als auch Keratinfasern. Amphotere Quaternierungsprodukte besitzen ein ausgezeichnetes Schaum- und Reinigungsvermögen. Beide Typen von Quaternierungsprodukten besitzen eine vorteilhafte hautkosmetische Verträglichkeit und biologische Abbaubarkeit.

### Tenside

Die Quaternierungsprodukte können mit weiteren oberflächenaktiven Stoffen abgemischt werden. Als oberflächenaktive Stoffe können nichtionische, anionische, kationische und/oder amphotere bzw. amphotere Tenside enthalten sein, deren Anteil an den Detergensgemischen üblicherweise etwa 50 bis 99 und vorzugsweise 70 bis 90 Gew.-% beträgt. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Ein besonderer Vorteil der kationischen Quaternierungsprodukte liegt dabei in der Tatsache, daß sie ausgesprochen aniontensidverträglich sind und mit diesen nicht ohne weiteres Salze bilden. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen.

### Wasch-, Spül-, Reinigungs- und Avivagemittel

Ein weiterer Gegenstand der Erfindung betrifft die Verwendung der quaternierten Fettsäureamidoaminethoxlate zur Herstellung von Wasch-, Spül-, Reinigungs- und Avivagemitteln, in denen sie in Mengen von 1 bis 50, vorzugsweise 5 bis 25 Gew.-% - bezogen auf die Mittel - enthalten sein können. Typische Beispiele sind flüssige bis pastöse Textilweichspülmittel, Handgeschirrspülmittel, maschinelle Geschirrspülmittel₁ Klarspülmittel, Universal-, Haushalts- und Sanitärreiniger sowie Wäscheweichspülmittel. Die Mittel können neben den bereits genannten Tensiden weitere typische Inhaltsstoffe wie beispielsweise Builder, Enzyme, Enzymstabilisatoren, Bleichmittel, optische Aufheller, Verdickungsmittel, Schauminhibitoren, Lösungsvermittler, anorganische Salze sowie Duft- und Farbstoffe aufweisen.

Geeignete **Builder** sind Zeolithe, Schichtsilicate, Phosphate sowie Ethylendiamintetraessigsäure, Nitrilotriessigsäure, Citronensäure sowie anorganische Phosphonsäuren` Unter den als Peroxy-**Bleichmittel** dienenden Verbindungen haben das Natriumperborat-Tetrahydrat und das Natriumperborat-Monohydrat eine besondere Bedeutung. Weitere Bleichmittel sind beispielsweise Peroxycarbonat, Citratperhydrate sowie H₂O₂-liefernde persaure Salze der Persäuren wie Perbenzoate, Peroxyphthalate oder Diperoxydodecandisäure. Sie werden üblicherweise in Mengen von 8 bis 25 Gew.-% eingesetzt. Bevorzugt ist der Einsatz von Natriumperborat-Monohydrat in Mengen von 10 bis 20 Gew.-% und insbesondere von 10 bis 15 Gew.-%. Durch seine Fähigkeit, unter Ausbildung des Tetrahydrats freies Wasser binden zu können , trägt es zur Erhöhung der Stabilität des Mittels bei.

Als **Verdickungsmittel** können beispielsweise gehärtetes Rizinusöl, Salze von langkettigen Fettsäuren, die vorzugsweise in Mengen von 0 bis 5 Gew.-% und insbesondere in Mengen von 0,5 bis 2 Gew.-%, beispielsweise Natrium-, Kalium-, Aluminium-, Magnesium- und Titan-Stearate oder die Natrium und/oder Kaliumsalze der Behensäure, sowie weitere polymere Verbindungen eingesetzt werden. Zu den letzten gehören bevorzugt Polyvinylpyrrolidon, Urethane und die Salze polymerer Polycarboxylate, beispielsweisehomopolymerer oder copolymerer Polyacrylate, Polymethacrylate und insbesondere Copolymere der Acrylsäure mit Maleinsäure, vorzugsweise solche aus 50 bis 10 Gew.-% Maleinsäure. Die relative Molekülmasse der Homopolymeren liegt im allgemeinen zwischen 1000 und 100000, die der Copolymeren zwischen 2000 und 200000, vorzugsweise zwischen 50000 bis 120000, bezogen auf die freie Säure. Insbesondere sind auch wasserlösliche Polyacrylate geeignet, die beispielsweise mit etwa 1% eines Polyallylethers der Sucrose quervernetzt sind und die eine relative Molekülmasse oberhalb 1000000 besitzen Beispiele hierfür sind unter dem Namen Carbopol® 940 und 941 erhältliche Polymere. Die quervernetzten Polyacrylate werden vorzugsweise in Mengen nicht über 1 Gew.-% besonders bevorzugt in Mengen von 0,2 bis 0,7 Gew.-% eingesetzt.

Als **Enzyme** kommen solche aus der Klasse der Proteasen, Lipase, Amylasen, Cellulasen bzw. deren Gemische in Frage. Besonders gut geeignet sind aus Bakterienstämmen oder Pilzen, wie Bacillus subtilis, Bacillus lichenformis und Strptomyces griseus gewonnene enzymatische Wirkstoffe. Vorzugsweise werden Proteasen vom Subtilisin-Typ und insbesondere Proteasen, die aus Bacillus lentes gewonnen werden, eingesetzt. Ihr Anteil kann etwa 0,2 bis 2 Gew.-% betragen. Die Enzyme können an Trägerstoffen adsorbiert oder in Hüllsubstanzen eingebettet sein, um sie gegen vorzeitige Zersetzung zu schützen.

Zusätzlich zu mono- und polyfunktionellen Alkoholen und Phosphonaten können die Mittel weitere **Enzymstabilisatoren** enthalten. Beispielsweise können 0,5 bis 1 Gew.-% Natriumformiat eingesetzt werden. Möglich ist auch der Einsatz von Proteasen, die mit löslichen Calciumsalzen und einem Calciumgehalt von vorzugsweise etwa 1,2 Gew.-%, bezogen auf das Enzym, stabilisiert sind. Besonders vorteilhaft ist jedoch der Einsatz von Borverbindungen, beispielsweise von Borsäure, Boroxid, Borax und anderen Alkalimetallboratenwie den Salzen der Orthoborsäure (H₃BO₃), der Metaborsäure (HBO₂) und der Pyroborsäure (Tetraborsäure H₂B₄O₇).

Beim Einsatz im maschinellen Waschverfahren kann es von Vorteil sein, den Mitteln übliche **Schauminhibitoren** zuzusetzen. Geeignete Schauminhibitoren enthalten beispielsweise bekannte Organoolysiloxane, Paraffine oder Wachse.

### Kosmetische und/oder pharmazeutische Zubereitungen

Kosmetische bzw. pharmazeutische Zubereitungen auf Basis der erfindungsgemäßen quaternierten Fettsäureamidoaminethoxylate, wie beispielsweise Haarshampoos, Haarlotionen, Schaumbäder, Cremes, Lotionen oder Salben, können ferner als weitere Hilfs- und Zusatzstoffe Ölkörper, Emulgatoren, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzflter, Insektenrepellentien, Selbstbräuner, Farb- und Duftstoffe enthalten.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, Ester von C₆-C₂₂-Fett-alkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate Guerbetcarbonate, Dialkylether, Siliconöle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxid-anlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentrimamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozuckerwie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

### Beispiel 1:

In einem 1-l-Dreihalskolben mit Rührer, Rückflußkühler und Tropftrichter wurden 540 g (2 Mol) partiell gehärtete Talgfettsäure und 1 g Hypophosphorsäure vorgelegt und auf 90°C erwärmt. Anschließend wurden der Mischung portionsweise 103 g (1 Mol) Diethylentriamin zugesetzt und auf 200°C erhitzt. Die Amidierung wurde solange durchgeführt, bis die Säurezahl unter einen Wert von 7 mg KOH/g abgesunken war. Danach wurden 589 g (1 Mol) des gebildeten Fettsäureamidoamins in einen Autoklaven überführt und bei einer Temperatur von 115°C und einem autogenen Druck von 1,5 bar mit 88 g (2 Mol) Ethylenoxid umgesetzt. 677 g (1 Mol) des resultierenden ethoxylierten Amidoamins wurden bei 50°C in 1000 ml Isopropylalkohol gelöst und dann bei 80°C mit 120 g (0,95 Mol) Dimethylsulfat quaterniert. Nach der Zugabe wurde die Mischung 2 h bei 80°C gerührt und dann abgekühlt.

### Beispiel 2

Beispiel 1 wurde wiederholt. Nach Absinken der Säurezahl auf unter 7 mg KOH/g wurde jedoch ein Vakuum von 15 mbar angelegt und die Reaktionsmischung weitere 2 bei diesen Bedingungen gerührt. Anschließend wurde die Mischung auf 80°C abgekühlt und zur Hydrolyse gebildeten Imidazolins 36 g (2 Mol) Wasser zugesetzt. Die Mischung wurde bei 80°C 8 h gerührt und dann das Wasser im Vakuum entfernt. Anschließend wurde das Reaktionsprodukt wie in Beispiel 1 beschrieben ethoxyliert und quaterniert.

### Beispiel 3

Beispiel 1 wurde wiederholt, jedoch das Ethoxylat in einer entsprechenden Menge Wasser dispergiert und anstelle von Dimethylsulfat mit 148 g (0,95 Mol) Natriumchloracetat quaterniert.

### Beispiel 4

Beispiel 2 wurde wiederholt, jedoch das Ethoxylat in einer entsprechenden Menge Wasser dispergiert und anstelle von Dimethylsulfat mit 148 g (0,95 Mol) Natriumchloracetat quaterniert.

## Patentansprüche

1. Quaternierte Fettsäureamidoaminethoxylate der Formel **(I),** 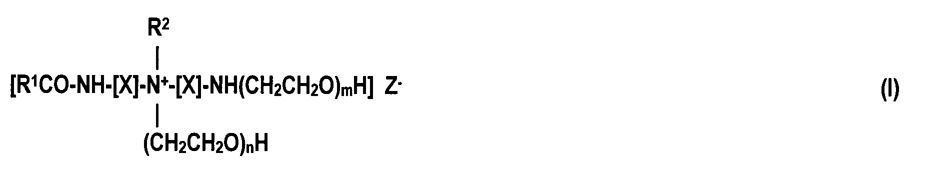 in der R¹CO für einen linearen oder verzweigten, gesättigten und/oder ungesättigten Acylrest mit 6 bis 22 Kohlenstoffatomen, R² für für einen Alkylrest mit 1 bis 4 Kohlenstoffatomen oder einen CH₂COOY-Rest, die Summe (m+n) für Zahlen von 1 bis 20 und q für Zahlen von 1 bis 5, X für eine lineare oder verzweigte Alkylengruppe mit 1 bis 6 Kohlenstoffatomen oder eine CH₂CH₂-(NH-CH₂CH₂)_{q}-Gruppe, Y für Wasserstoff oder ein Alkalimetall und Z für Halogenid oder Alkylsulfat steht.

2. Verfahren zur Herstellung von quaternierten Fettsäureamidoaminethoxylaten der Formel **(I)**, 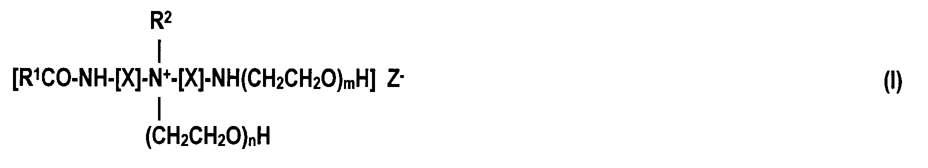 in der R¹CO für einen linearen oder verzweigten, gesättigten und/oder ungesättigten Acylrest mit 6 bis 22 Kohlenstoffatomen, R² für für einen Alkylrest mit 1 bis 4 Kohlenstoffatomen oder einen CH₂COOY-Rest, die Summe (m+n) für Zahlen von 1 bis 20 und q für Zahlen von 1 bis 5, X für eine lineare oder verzweigte Alkylengruppe mit 1 bis 6 Kohlenstoffatomen oder eine CH₂CH₂-(NH-CH₂CH₂)_{q}-Gruppe, Y für Wasserstoff oder ein Alkalimetall und Z für Halogenid oder Alkylsulfat steht, bei dem man
(a) Fettsäuren mit Di- bzw. Oligoaminen kondensiert,
(b) die resultierenden Fettsäureamidoamine an sich bekannter Weise mit Ethylenoxid umsetzt
und
(c) die resultierenden ethoxylierten Fettsäureamidoamine anschließend in an sich bekannter Weise mit Alkylierungsmitteln quaterniert.

3. Verfahren nach Anspruch 2, dadurch gekennzeichnet, daß man Fettsäuren der Formel **(II)** einsetzt, 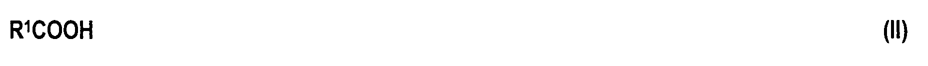 in der R¹CO die oben angegebene Bedeutung besitzt.

4. Verfahren nach den Ansprüchen 2 und 3, dadurch gekennzeichnet, daß man Di- bzw. Oligoamine der Formel **(III)** einsetzt, 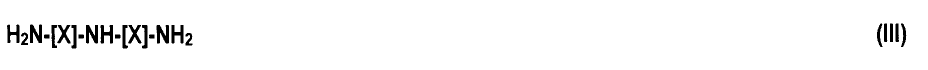 in der X die oben angegebene Bedeutung besitzt.

5. Verfahren nach den Ansprüchen 2 bis 4, **dadurch gekennzeichnet**, daß man die Fettsäuren und die Amine im molaren Verhältnis 1,3 : 1 bis 2,3 : 1 einsetzt.

6. Verfahren nach den Ansprüchen 2 bis 5, **dadurch gekennzeichnet**, daß man die Fettsäureamidoamine mit durchschnittlich 1 bis 20 Mol Ethylenoxid pro Mol Amid umsetzt.

7. Verfahren nach den Ansprüchen 2 bis 6, **dadurch gekennzeichnet**, daß man die ethoxylierten Fettsäureamidoamine mit Alkylierungsmitteln quaterniert, die ausgesucht sind aus der Gruppe, die gebildet wird, von Methylhalogeniden, Dialkylsulfaten und Halogencarbonsäuren bzw. deren Alkalisalzen.

8. Verfahren nach den Ansprüchen 2 bis 7, daß man sekundäre Amidoamine durch Imidazolinbildung und nachfolge Ringöffnung zerstört.

9. Verwendung von quaternierten Fettsäureamidoaminethoxylaten nach Anspruch 1 zur Herstellung von Wasch-, Spül-, Reinigungs- und Avivagemitteln.

10. Verwendung von quaternierten Fettsäureamidoaminethoxylaten nach Anspruch 1 zur Herstellung von kosmetischen und/oder pharmazeutischen Zubereitungen.
